# EUROPEAN PATENT SPECIFICATION

(11) **EP 2 843 404 B1**
(45) Date of publication and mention of the grant of the patent: **11.10.2017**
(21) Application number: 14180199.3
(22) Date of filing: 07.08.2014
(51) Int. Cl.: G01N 33/28, F02M 37/00, F02M 37/22, F02D 33/00

(54) **Fuel property detection apparatus for construction machine**
Brennstoffeigenschaftsnachweisvorrichtung für eine Baumaschine
Appareil de détection de propriété de carburant pour machine de construction

(30) Priority: 27.08.2013 JP 2013175419
(43) Date of publication of application: 04.03.2015
(73) Proprietor: Kobelco Construction Machinery Co., Ltd., Hiroshima 731-5161 (JP)
(72) Inventor: Li, Bocheng, Hiroshima-shi,, Hiroshima 731-5161 (JP); Kita, Tomotaka, Hiroshima-shi,, Hiroshima 731-5161 (JP)
(74) Representative: TBK

(56) References cited:
- EP-A1- 2 495 542
- JP-A- H1 113 568
- US-A- 4 031 864
- US-A1- 2009 303 466
- US-A1- 2012 126 835

## Description

### Background of Invention

### Technical field

The present invention relates to a fuel property detection apparatus for detecting a property of fuel supplied to an engine in a hydraulic excavator or other construction machines in order to determine the adequacy of the fuel.

### Background art

There have been known apparatuses that each are adapted for detecting a property of fuel supplied to an engine provided in a hydraulic excavator or other construction machines in order to prevent a breakage of the engine or generation of inferior exhaust gas due to an inadequacy of fuel used in the engine. The apparatus is adapted to detect, for example, a physical quantity such as kinematic viscosity and density, or a chemical property.

Japanese Unexamined Patent Publication No. 2011-94549 (Patent Document 1) discloses a so-called sub-tank type apparatus. This apparatus includes a small sub-tank disposed in a fuel supply line connecting an engine with a fuel tank, and a sensor disposed on a bottom section of the sub-tank. The sensor detects a property of the fuel under a condition of no flow velocity and a stabilized flow rate of the fuel in the sub-tank. Also, Japanese Unexamined Patent Publication No. 2008-261812 (Patent Document 2) discloses a technique of detecting a fuel property inside a fuel tank.

The sub-tank type apparatus disclosed in Patent Document 1 can be easily mounted in an existing construction machine in an additional manner as compared to the apparatus disclosed in Patent Document 2, and is further capable of accurate and stable detection of fuel property; however, there is a likelihood that the sensor comes into contact with water contained in the fuel (or water separated from the fuel) to thereby decrease detection accuracy. Furthermore, water has the greater specific gravity than fuel (for example, light oil), and is likely to accumulate on the bottom of the sub-tank, thus significantly decreasing the detection accuracy of the sensor particularly in an apparatus where the sensor is disposed on the bottom of the fuel tank, thus disabling the apparatus from detecting fuel property Besides, even a sensor disposed on a location other than the bottom of the fuel tank cannot be completely prevented from contact with water, because the water having accumulated on the bottom of the fuel tank can be moved in the sub-tank by the flow of fuel or stirring currents accompanied by this flow.

EP 2 495 542 A1 discloses a fuel property detection apparatus having the features of the preamble of claim 1. The apparatus has a sub-tank and a sensor for detecting a property of the fuel in the sub-tank. The sub-tank has one fuel-inlet and one fuel-outlet.

US 4 031 864 A discloses a multiple fuel supply system for an internal combustion engine. The system comprises a fuel tank. The fuel tank has an inlet line at an upper side of the fuel tank, and an outlet line at a lateral side of the fuel tank. At a lower side of the fuel tank there is arranged a density measuring device. The outlet line extends from an inner part of the fuel tank to the outside thereof through a side wall of the fuel tank.

### Summary of the Invention

An object of the present invention is to provide an apparatus for detecting a property of fuel used in a construction machine including an engine and a fuel tank, the apparatus being capable of detecting a property of fuel securely and with high accuracy, with use of a sub-tank.

The object is solved by a fuel property detection apparatus having the features of claim 1. Further developments are subject matter of the dependent claims.

### Brief description of drawings

Fig. 1 is a schematic diagram illustrating a system including a fuel property detection apparatus according to an embodiment of the present invention.
Fig. 2 is a cross sectional view illustrating a configuration of a sub-tank of the apparatus and flows of fuel.
Fig. 3 is a cross sectional view taken along the line III-III in Fig. 2.
Fig. 4 is an enlarged view showing a bottom section, a fuel introducing part, and a lower fuel discharging part of the sub-tank illustrated in Fig. 2.
Fig. 5 is an enlarged view showing an upper section and an upper fuel discharging part of the sub-tank illustrated in Fig. 2.

### Embodiment of the Invention

An embodiment of the present invention is described below with reference to the drawings.

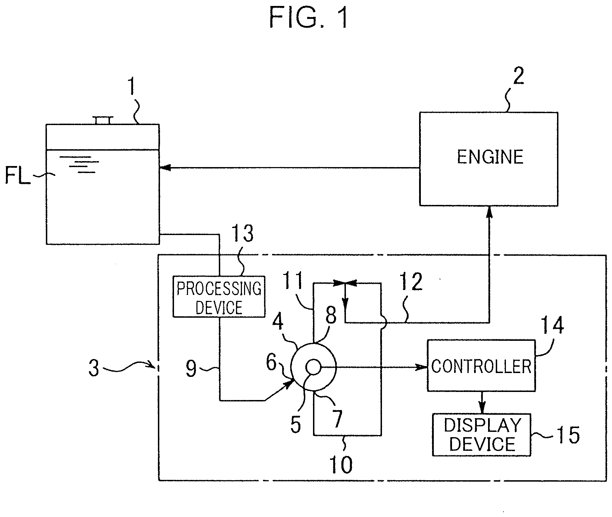

Fig. 1 illustrates a fuel property detection apparatus according to the embodiment. The apparatus is provided in a construction machine, as illustrated in Fig. 1, including an engine 2 and a fuel tank 1 for containing fuel FL to be used for the engine 2. The apparatus constitutes at least a part of a fuel supply line 3 for supplying the fuel FL from the fuel tank 1 to the engine 2, and performs detection of a property of the supplied fuel FL. The apparatus includes a sub-tank 4, a sensor 5, a fuel introducing pipe 9, and a fuel discharger including a bottom fuel discharging pipe 10, an upper fuel discharging pipe 11, and a junction pipe 12.

The sub-tank 4 is disposed between the engine 2 and the fuel tank 1 to contain the fuel FL to be supplied to the engine 2 from the fuel tank 1. The sub-tank 4 is a small-sized tank having a capacity smaller than that of the fuel tank 1.

The sensor 5 is provided in the sub-tank 4 to detect a property of the fuel FL inside the sub-tank 4. Specifically, the sensor generates a detection signal in the form of an electric signal indicative of a property of the fuel FL. The property to be detected may be a physical quantity such as kinematic density or density, or may be a chemical property.

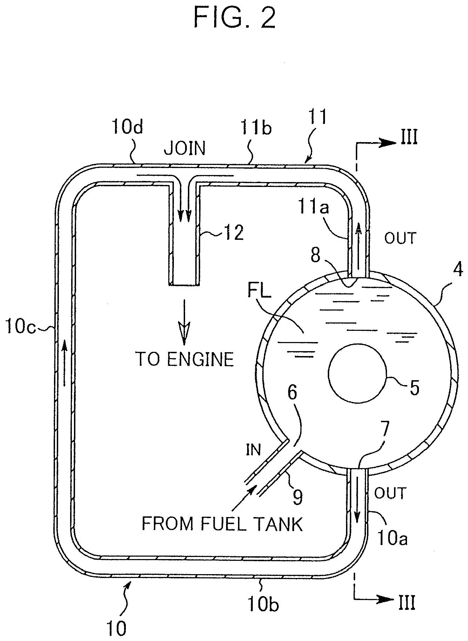

As also illustrated in Fig. 2 to Fig. 5, the sub-tank 4 according to the embodiment has a fuel inlet 6, a bottom fuel outlet 7, and an upper fuel outlet 8. Each of these is an opening which allows the fuel FL to pass through. The bottom fuel outlet 7 is formed in the bottom section of the sub-tank 4, and the upper fuel outlet 8 is formed in a top section of the sub-tank 4. In this embodiment, the fuel inlet 6 is formed at a location which is in a lower section of the sub-tank 4 but slightly upper of the bottom fuel outlet 7.

The fuel introducing pipe 9 is provided between the fuel tank 1 and the sub-tank 4 to allow the fuel FL to be supplied from the fuel tank 1 to the sub-tank 4 through the fuel introducing pipe 9. The fuel introducing pipe 9 has a downstream end connected to the fuel inlet 6, through which the fuel FL is introduced into the sub-tank 4. The bottom fuel discharging pipe 10 has an upstream end connected to the bottom fuel outlet 7, and the upper fuel discharging pipe 11 has an upstream end connected to the upper fuel outlet 8. Accordingly, the fuel FL in the sub-tank 4 can be discharged to the engine 2 from the bottom section of the sub-tank 4 via the bottom fuel outlet 7 and the bottom fuel discharging pipe 10, and can also be discharged to the engine 2 from the upper section of the sub-tank 4 via the upper fuel outlet 8 and the upper fuel discharging pipe 11.

The bottom fuel discharging pipe 10 and the upper fuel discharging pipe 11 have respective downstream ends which are connected to a common junction pipe 12 so as to join to each other at the junction pipe 12. The junction pipe 12 has an upstream end connected to the fuel discharging pipes 10 and 11, and a downstream end connected to the engine 2.

In the embodiment, as shown in Fig. 1, a processing device 13 is provided in an intermediate location of the fuel introducing pipe 9. The processing device 13 comprises a water separator, a filter, and like members. Alternatively, the processing device 13 may be provided in the junction pipe 12.

Furthermore, the fuel supply line 3 according to the embodiment includes a fuel pump (not illustrated). The fuel pump has a pressurizing function for causing the fuel FL in the fuel tank 1 to flow to the engine 2 through the fuel supply line 3.

The sub-tank 4, provided in the fuel supply line 3 as described above, is able to decrease the flow velocity of the fuel FL in the sub-tank 4 and stabilize flow rate thereof. The sensor 5, thus generating a detection signal regarding a property of the fuel FL in the sub-tank 4 under such a condition that the fuel FL has the decreased flow velocity and the stabilized flow rate, brings about the basic effect of securing the time required for detecting a fuel property and the stabilized flow rate.

The sub-tank 4 can be favorably mounted on a suitable part of the construction machine, for example on an upper frame of an upper slewing body in the case of a hydraulic excavator. That is because: (I) as the position on which the sub-tank 4 is to be provided, any advantageous position in terms of space and mountability can be selected among positions between the fuel tank 1 and the engine 2, and (II) the sub-tank 4 can be a small-sized tank because it only has to have a capacity enough to decrease the flow velocity to one required for detecting a property of the fuel FL. This apparatus thus allows the accuracy and the stability in the detection of a fuel property to be basically improved while employing the sub-tank type capable of being additionally installed onto an existing machine and having good mountability.

In the example illustrated in Fig. 1, the detection signal generated by the sensor 5 is inputted into a controller 14. The controller 14 determines adequacy of the fuel FL based on the detection signal, and causes a display device 15 to perform display, warning, and the like.

Next described in details are configurations of the sub-tank 4 and its related parts.

In the embodiment, the sub-tank 4 has a cylindrical peripheral wall having a horizontal central axis. The fuel inlet 6 is formed in a substantially center location in a longitudinal direction, i.e., the axis direction, of the peripheral wall, and is obliquely downwardly directed toward the outside of the sub-tank 4 in axial view, as illustrated in Fig. 2. The bottom fuel outlet 7 is vertically downwardly directed toward the outside of the sub-tank 4, and the upper fuel outlet 8 is vertically upwardly directed toward the outside of the sub-tank 4.

As illustrated in Fig. 2 and other drawings, the bottom fuel discharging pipe 10 according to the embodiment has an upstream vertical section 10a, an intermediate horizontal section 10b, a downstream vertical section 10c and a downstream horizontal section 10d. The upstream vertical section 10a extends vertically, and has an upper end connected to the bottom fuel outlet 7. The intermediate horizontal section 10b extends horizontally from a lower end of the upstream vertical section 10a. The downstream vertical section 10c extends vertically upwardly from one end of opposite ends of the intermediate horizontal section 10b, the one end being opposite to the intermediate horizontal section 10b, the other end connected to the upstream vertical section 10a. The downstream horizontal section 10d extends horizontally in such a direction as to come closer to the sub-tank 4 from an upper end of the downstream vertical section 10c.

The upper fuel discharging pipe 11 has a vertical section 11a and a horizontal section 11b. The vertical section 11a extends vertically and has a lower end connected to the upper fuel outlet 8. The horizontal section 11b extends horizontally in such a direction as to come closer to the downstream horizontal section 10d of the bottom fuel discharging pipe 10 from an upper end of the vertical section 11a.

The downstream horizontal section 10d and the horizontal section 11b have respective downstream ends which are connected to an upstream end of the junction pipe 12 so as to join to each other at the common junction pipe 12. This connection enables the fuel FL flowing in the upper fuel discharging pipe 11 to join to the fuel FL discharged through the bottom fuel discharging pipe 10 at the highest part of the upper fuel discharging pipe 11.

The fuel introducing pipe 9 and the fuel discharging pipes 10 and 11 are constituted by, for example, an inflow connector steel cylinder and outflow connecting steel cylinders connected to the sub-tank 4, and hoses connected to the steel cylinders, respectively. Meanwhile, in Fig. 2, each of the pipes 9 to 11 is indicated just as single pipe for simplification. Similarly, the junction pipe 12, which is constituted by, for example, a T-shaped joint provided in the joining part and a hose connected to the joint, is indicated as one pipe in Fig. 2.

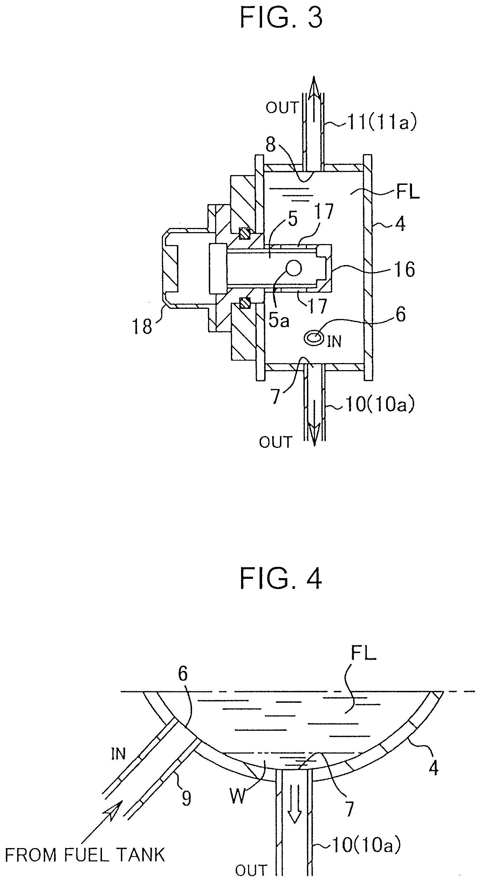

Fig. 3 shows an example of a configuration for attachment of the sensor 5. The sensor 5 has a sensing section 5a and is attached to one of the longitudinally opposite walls of the sub-tank 4 so as to allow the sensing section 5a to make contact with the fuel FL in the sub-tank 4 at a middle position (a center or a position near to the center) in the height direction of the sub-tank 4 and a middle position (a center or a position near to the center) in the longitudinal direction of the sub-tank 4.

The sensor 5 according to the embodiment is attached to the sub-tank 4 while being contained in a sensor case 16 as shown in Fig. 3. The sensor case 16 has upper and lower fuel passage holes 17 that bring the inside and outside of the sensor case 16 into communication with each other. The fuel passage holes 17 allows the fuel FL in the sub-tank 4 to enter inside the sensor case 16 through the fuel passage hole 17 to enable the sensor 5 to detect the property of the fuel FL. Besides, the sensor case 16 has a sensor cover 18, which covers an inner space of the sensor case 16 outside the sub-tank 4.

In the fuel property detection apparatus, the fuel FL in the fuel tank 1 is introduced into the sub-tank 4 through the fuel introducing pipe 9 and the fuel inlet 6. Only a part of the inflowing fuel FL is held in the sub-tank 4, and the other part exceeding the certain amount is discharged from the sub-tank 4 to the engine 2 through the bottom fuel outlet 7 and the fuel discharging pipe 10 and through the upper fuel outlet 8 and the fuel discharging pipe 11. The sensor 5 comes into contact with the fuel FL contained in the sub-tank 4 to generate a detection signal concerning the property of the fuel FL.

In the case where the fuel FL contains a bit of water, for example, the water having failed to be removed in the processing device 13, the bit of water, which has a greater specific gravity than the fuel FL, is liable to gradually separate from the fuel FL and accumulate on the bottom section of the sub-tank 4. In this situation, without the bottom fuel outlet 7 and the bottom fuel discharging pipe 10, the water could accumulate on the bottom section of the sub-tank as illustrated in Fig. 4 and the accumulated water might be moved in the sub-tank together with fuel by the flow of the fuel FL within the sub-tank or stirring currents accompanied by the flow of the fuel, thus coming into contact with a sensor to give bad effects to the detection of the sensor.

In contrast, in the above-described apparatus with the bottom fuel outlet 7 in the bottom section, that is, the lowest part, of the sub-tank 4, and the bottom fuel discharging pipe 10 connected to the bottom fuel outlet 7, the water is forcibly discharged to the downstream side through the bottom fuel discharging pipe 10 by the kinetic energy of the outflowing fuel FL through the lower fuel outlet 7, specifically, a flowing energy due to the pressurization by the fuel pump and a falling energy due to the self-weight of the fuel FL. Water is thus prevented from accumulating on the bottom section of the sub-tank 4. Even if having accumulated, the water is efficiently discharged from the sub-tank 4. This makes it possible to reduce the amount of water existing in the sub-tank 4 to minimize the influence of water on the detection of the sensor 5.

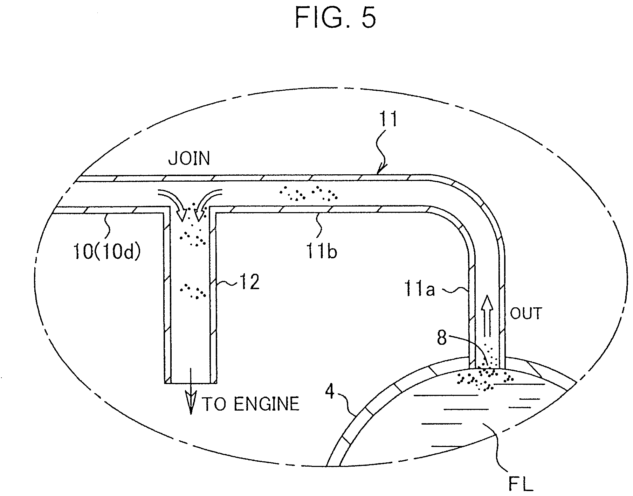

On the other hand, a bit of air contained in the fuel FL rises in the sub-tank 4, opposite to the water. If air accumulating on the top section of the sub-tank 4, the air would come into contact with the sensor 5 due to the flowing and stirring currents of the fuel FL to thereby cause bad effects to the property detection of the fuel FL. However, in the apparatus according to the embodiment, the sub-tank 4, having the upper fuel outlet 8 in its top section as well to which the upper fuel discharging pipe 11 is connected, allows the air accumulating on the top section of the sub-tank 4 to be flown out to the downstream side by the flowing energy of the fuel FL, as schematically shown by black dots in Fig. 5. In the embodiment, the bad influence of air to the detecting performance is thus minimized.

Furthermore, the discharged fuel FL flowing through the upper fuel discharging pipe 11 according to the embodiment joins to the discharged fuel FL flowing in the bottom fuel discharging pipe 10 at the highest part of the upper fuel discharging pipe 11 by the way of the junction pipe 12, thus enabling the air to flow into the junction pipe 12 owing to the joining energy. This prevents the air from accumulation in the upper fuel discharging pipe 11 to improve the flow of the fuel FL, thus allowing the air discharging performance to be enhanced.

Moreover, in this embodiment, the contact of the sensing section 5a of the sensor 5 with the fuel FL at a vertically middle section of the sub-tank 4, that is, at the section having less respective distributions of water and air than the bottom and top sections of the sub-tank 4, allows the detection accuracy to be enhanced.

For the above-described reasons, the fuel property detection apparatus can suppress the bad influences of water and air securely to thereby carry out detection of a fuel property in a high accuracy.

The present invention is not limited to the embodiment described above, including, for example, the following embodiments.
(1) The fuel inlet 6 of the sub-tank 4, which is formed at the obliquely lower left portion as shown in Fig. 2, is also permitted to be formed at a position laterally symmetrical to the obliquely lower left position, that is, at an obliquely lower right position, at a position vertically symmetrical to the obliquely lower left position or the obliquely lower right position, that is, at an obliquely upper left position or an obliquely upper right position, or at either a left or right position in a middle part of the vertical length of the sub-tank 4. Alternatively, a plurality of fuel inlets 6 may be formed at respective plural positions.
(2) The shape of a sub-tank is not limited to that shown in the above embodiment, according to the present invention. In the viewpoint of facilitating the discharge of water and air owing to easy restriction of an area for collecting them, it is preferable to provide a sub-tank having a cylindrical shape extending horizontally as the sub-tank 4 illustrated in Fig. 2 and other drawings; a sub-tank according to the present invention, however, may have a cylindrical shape with a vertical axis or a rectangular-parallelepiped shape.
(3) The position of the sensor is not limited to that shown in the above embodiment, according the present invention. The sensor according to the present invention may be provided at a position lower or upper than the middle of the vertical length of the sub-tank, or at a position offset longitudinally of the sub-tank.

As described above, the present invention provides an apparatus which is used for a construction machine including an engine and a fuel tank to detect a property of fuel and capable of detecting a property of fuel securely and with high accuracy with use of a sub-tank. Specifically, the apparatus is to be provided on a construction machine including an engine and a fuel tank for containing fuel for supply to the engine to detect a property of fuel, the apparatus comprising: a sub-tank provided between the engine and the fuel tank to contain fuel to be supplied from the fuel tank to the engine; a fuel introducing pipe provided between the sub-tank and the fuel tank to allow fuel to be introduced from the fuel tank to the sub-tank through the introducing pipe; a fuel discharger provided between the sub-tank and the engine to allow fuel to be discharged from the sub-tank to the engine through the discharger; and a sensor provided in the sub-tank to generate a detection signal concerning a property of the fuel in the sub-tank. The sub-tank has a bottom section having a bottom fuel outlet for discharging fuel from the bottom section. The fuel discharger includes a bottom fuel discharging pipe connected to the bottom fuel outlet.

In the apparatus, the water which has accumulated or tends to stay on the bottom section of the sub-tank is forcibly discharged to the downstream side by a kinetic energy possessed by the outflowing fuel from the bottom section of the sub-tank, specifically, a flowing energy caused by the applied pressure by a fuel pump and a falling energy caused by the self-weight of the fuel, thereby accomplishing efficient discharge of water from the sub-tank: this makes it possible to effectively suppress the influences of the water in the sub-tank to the detection.

It is preferable that the sub-tank further has an upper fuel outlet positioned in a top section of the sub-tank, and the fuel discharger further includes an upper fuel discharging pipe connected to the upper fuel outlet. The air contained in the fuel is likely to accumulate on a highest part of the sub-tank, opposite to water, and, if accumulating, the air could be moved in the fuel by the flowing and stirring currents of the fuel to come into contact with the sensor, thus causing bad influences to the fuel property detection, similarly to water; however, the upper fuel outlet and the upper fuel discharging pipe connected to the upper fuel outlet allows the accumulated air on the highest part of the sub-tank to be flown out to the downstream side due to the flowing energy of the fuel to be discharged from the sub-tank, thus also making it possible to suppress the bad influences of air to the detection performance.

In this arrangement, it is preferable that the fuel discharger further includes a junction pipe connected to the bottom fuel discharging pipe and the upper fuel discharging pipe to allow the fuel flowing through the bottom fuel discharging pipe to join to the fuel flowing through the upper fuel discharging pipe at the junction pipe and flow to the engine, the upper fuel discharging pipe being connected to the junction pipe at a highest part of the upper fuel discharging pipe. The respective fuels flowing through the bottom fuel discharging pipe and through the upper fuel discharging pipe can join to each other at the highest part of the upper fuel discharging pipe, thereby enabling the air to be flown into the junction pipe by the joining energy. This can prevent the air from accumulating in the upper fuel discharging pipe, and improve the flow of fuel, thus allowing the air discharging performance and further detection accuracy to be enhanced.

It is preferable that the sensor includes a sensing section and is provided at a vertically middle part of the sub-tank so as to allow the sensing section to make contact with the fuel in the sub-tank. This contact of the sensing section with the fuel at the vertically middle part of the sub-tank, where the distribution of water and air is less than the bottom and top sections of the sub-tank, enables the detection accuracy to be increased.

Provided is an apparatus for detecting property of fuel in a construction machine, with use of a sub-tank, the apparatus being capable of minimizing an influence on the detection given by water contained in fuel. The apparatus includes a sub-tank (4) between an engine (2) and a fuel tank (1) of the construction machine, a fuel introducing pipe (9) through which fuel is introduced to the sub-tank (4) from the fuel tank (1), a fuel discharger through which fuel in the sub-tank (4) is discharged to the engine (2), and a sensor (5). The sub-tank (4) has a bottom section having a bottom fuel outlet (7) for discharging fuel from the bottom section. The fuel discharger includes a bottom fuel discharging pipe (10) connected to the bottom fuel outlet (7).

## Claims

1. A fuel property detection apparatus to be provided on a construction machine including an engine (2) and a fuel tank (1) for containing fuel (FL) to be supplied to the engine (2), to detect a property of fuel (FL), the apparatus comprising:
a sub-tank (4) provided between the engine (2) and the fuel tank (1) to contain fuel (FL) to be supplied from the fuel tank (1) to the engine (2);
a fuel introducing pipe (9) provided between the sub-tank (4) and the fuel tank (1) to allow fuel (FL) to be introduced from the fuel tank (1) to the sub-tank (4) through the fuel introducing pipe (9);
a fuel discharger provided between the sub-tank (4) and the engine (2) to allow fuel to be discharged from the sub-tank (4) to the engine (2) through the fuel discharger; and
a sensor (5) provided in the sub-tank (4) to generate a detection signal concerning a property of the fuel in the sub-tank (4), wherein
the sub-tank (4) has a bottom section that is the lowest part of the sub-tank (4), and a top section of the sub-tank (4),
the top section has an upper fuel outlet (8) for discharging fuel (FL) from the top section, **characterized in that**
the bottom section has a bottom fuel outlet (7) for discharging fuel from the bottom section,
the fuel discharger includes a bottom fuel discharging pipe (10) connected to the bottom fuel outlet (7) and an upper fuel discharging pipe (11) connected to the upper fuel outlet (8), wherein
the sensor (5) includes a sensing section and is provided in the sub-tank (4) so as to allow the sensing section to make contact with the fuel in the sub-tank (4) at a vertically middle part of the sub-tank (4).

2. The fuel property detection apparatus for a construction machine as set forth in claim 1, wherein the fuel discharger further includes a junction pipe (12) connected to the bottom fuel discharging pipe (10) and the upper fuel discharging pipe (11) to allow the fuel flowing in the bottom fuel discharging pipe (10) to join to the fuel flowing in the upper fuel discharging pipe (11) and flow to the engine (2), the upper fuel discharging pipe (11) being connected to the junction pipe (12) at a highest part of the upper fuel discharging pipe (11).

3. The fuel property detection apparatus for a construction machine as set forth in claim 1 or 2, wherein the vertically middle part includes a center of the sub-tank (4).

## Patentansprüche

1. Kraftstoffeigenschaftserfassungsgerät, das an einer Baumaschine vorzusehen ist, die eine Kraftmaschine (2) und einen Kraftstofftank (1) zum Aufnehmen von zu der Kraftmaschine (2) zuzuführendem Kraftstoff (FL) aufweist, um eine Eigenschaft des Kraftstoffs (FL) zu erfassen, wobei das Gerät Folgendes aufweist:
einen Nebentank (4), der zwischen der Kraftmaschine (2) und dem Kraftstofftank (1) vorgesehen ist, um von dem Kraftstofftank (1) zu der Kraftmaschine (2) zuzuführenden Kraftstoff (FL) aufzunehmen;
ein Kraftstoffeinbringrohr (9), das zwischen dem Nebentank (4) und dem Kraftstofftank (1) vorgesehen ist, um zu ermöglichen, dass Kraftstoff von dem Kraftstofftank (1) zu dem Nebentank (4) durch das Kraftstoffeinbringrohr (9) eingebracht wird;
eine Kraftstoffabgabeeinrichtung, die zwischen dem Nebentank (4) und der Kraftmaschine (2) vorgesehen ist, um zu ermöglichen, dass Kraftstoff von dem Nebentank (4) zu der Kraftmaschine (2) durch die Kraftstoffabgabeeinrichtung abgegeben wird; und
einen Sensor (5), der in dem Nebentank (4) vorgesehen ist, um ein Erfassungssignal betreffend einer Eigenschaft des Kraftstoffs in dem Nebentank (4) zu erzeugen, wobei
der Nebentank (4) einen unteren Abschnitt hat, der der niedrigste Teil des Nebentanks (4) ist, und einen oberen Abschnitt des Nebentanks (4) hat,
der obere Abschnitt einen oberen Kraftstoffauslass (8) zum Abgeben von Kraftstoff (FL) von dem oberen Abschnitt hat, **dadurch gekennzeichnet, dass**
der untere Abschnitt einen unteren Kraftstoffauslass (7) zum Abgeben von Kraftstoff von dem unteren Abschnitt hat,
die Kraftstoffabgabeeinrichtung ein unteres Kraftstoffabgaberohr (10) hat, das mit dem unteren Kraftstoffauslass (7) und einem oberen Kraftstoffabgaberohr (11) verbunden ist, welches mit dem oberen Kraftstoffauslass (8) verbunden ist, wobei
der Sensor (5) einen Erfassungsabschnitt aufweist und in dem Nebentank (4) vorgesehen ist, um es dem Erfassungsabschnitt zu ermöglichen, mit dem Kraftstoff in dem Nebentank (4) an einem in Vertikalrichtung mittleren Teil des Nebentanks (4) einen Kontakt einzugehen.

2. Kraftstoffeigenschaftserfassungsgerät für eine Baumaschine gemäß Anspruch 1, wobei die Kraftstoffabgabeeinrichtung ferner ein Abzweigungsrohr (12) hat, das mit dem unteren Kraftstoffabgaberohr (10) und dem oberen Kraftstoffabgaberohr (11) verbunden ist, um es dem in dem unteren Kraftstoffabgaberohr (10) strömenden Kraftstoff zu ermöglichen, mit dem in dem oberen Kraftstoffabgaberohr (11) strömenden Kraftstoff zusammenzufließen und zu der Kraftmaschine (2) zu strömen, wobei das obere Kraftstoffabgaberohr (11) an einem höchsten Teil des oberen Kraftstoffabgaberohrs (11) mit dem Abzweigungsrohr (12) verbunden ist.

3. Kraftstoffeigenschaftserfassungsgerät für eine Baumaschine gemäß Anspruch 1 oder 2, wobei der in Vertikalrichtung mittlere Teil eine Mitte des Nebentanks (4) aufweist.

## Revendications

1. Appareil de détection de propriété de carburant à prévoir sur une machine de construction comprenant un moteur (2) et un réservoir de carburant (1) pour contenir le carburant (FL) à amener au moteur (2), pour détecter une propriété du carburant (FL), l'appareil comprenant :
un sous-réservoir (4) prévu entre le moteur (2) et le réservoir de carburant (1) pour contenir le carburant (FL) à amener du réservoir de carburant (1) au moteur (2) ;
un tuyau d'introduction de carburant (9) prévu entre le sous-réservoir (4) et le réservoir de carburant (1) pour permettre d'introduire le carburant (FL) du réservoir de carburant (1) au sous-réservoir (4) en passant par le tuyau d'introduction de carburant (9),
un dispositif de décharge de carburant prévu entre le sous-réservoir (4) et le moteur (2) pour permettre la décharge du carburant du sous-réservoir (4) au moteur (2) par le biais du dispositif de décharge de carburant ; et
un capteur (5) prévu dans le sous-réservoir (4) pour générer un signal de détection concernant une propriété du carburant dans le sous-réservoir (4), dans lequel :
le sous-réservoir (4) a une section inférieure qui est la partie la plus basse du sous-réservoir (4) et une section supérieure du sous-réservoir (4),
la section supérieure a une sortie de carburant supérieure (8) pour décharger le carburant (FL) par la section supérieure, caractérisé en ce qui :
la section inférieure a une sortie de carburant inférieure (7) pour décharger le carburant de la section inférieure,
le dispositif de décharge de carburant comprend un tuyau de décharge de carburant inférieur (10) raccordé à la sortie de carburant inférieure (7) et un tuyau de décharge de carburant supérieur (11) raccordé à la sortie de carburant supérieure (8), dans lequel :
le capteur (5) comprend une section de détection et est prévu dans le sous-réservoir (4) pour permettre à la section de détection d'établir le contact avec le carburant dans le sous-réservoir (4) au niveau d'une partie verticalement centrale du sous-réservoir (4).

2. Appareil de détection de propriété de carburant pour une machine de construction selon la revendication 1, dans lequel le dispositif de décharge de carburant comprend en outre un tuyau de jonction (12) raccordé au tuyau de décharge de carburant inférieur (10) et au tuyau de décharge de carburant supérieur (11) pour permettre au carburant s'écoulant dans le tuyau de décharge de carburant inférieur (10) de rejoindre le carburant s'écoulant dans le tuyau de décharge de carburant supérieur (11) et s'écouler vers le moteur (2), le tuyau de décharge de carburant supérieur (11) étant raccordé au tuyau de jonction (12) au niveau de la partie la plus haute du tuyau de décharge de carburant supérieur (11).

3. Appareil de détection de propriété de carburant pour une machine de construction selon la revendication 1 ou 2, dans lequel la partie verticalement centrale comprend un centre du sous-réservoir (4).
